# EUROPEAN PATENT SPECIFICATION

(11) **EP 3 810 043 B1**
(45) Date of publication and mention of the grant of the patent: **30.11.2022**
(21) Application number: 20703924.9
(22) Date of filing: 17.02.2020
(51) Int. Cl.: A61F 5/00, A61F 2/64, A61F 5/01

(54) **BIAXIAL POLYCEMTRIC ARTICULATED JOINT FOR ORTHOPAEDIC ORTHOSES OR BRACES**
BIAXIALE POLYZENTRISCHE GELENKVERBINDUNG FÜR ORTHOPÄDISCHE ORTHESEN ODER BANDAGEN
ARTICULATION POLYCENTRIQUE BIAXIALE POUR LES ORTHÈSES OU APPAREILS ORTHOPÉDIQUES

(30) Priority: 22.02.2019 IT 201900002605
(43) Date of publication of application: 28.04.2021
(73) Proprietor: F.G.P. S.R.L., 37062 Villafranca Di Verona (Verona) (IT)
(72) Inventor: FERRIGOLO, Moreno, 37062 Villafranca di Verona (Verona) (IT); TURRINI, Alberto, 37062 Villafranca di Verona (Verona) (IT)
(74) Representative: Sandri, Sandro
(86) International application number: PCT/IB2020/051305
(87) International publication number: WO 2020/170111

(56) References cited:
- WO-A1-01/21114
- US-A- 5 443 444
- US-A1- 2004 068 215
- US-A1- 2006 287 624
- US-A1- 2018 036 159

## Description

### FIELD OF APPLICATION

The present invention, as defined in the claims, relates to a biaxial polycentric articulated joint for orthopaedic orthoses or braces designed for rehabilitation of joints of the human body, such as the knee, the elbow, the ankle, the shoulder or the like, whose particular feature is that it is provided with a means for adjusting the varus-valgus angle and locking it in position.

The present solution envisages providing a brace comprising a means adapted to correct some problems of knee or elbow joints or other joints, mainly tied to the problem of varus or valgus.

The two uprights or bars making up a traditional polycentric articulated joint are connected to each other thanks to a circular toothed profile present on each bar and the fact that the toothed profile sets them into a relative, synchronous motion with respect to each other and that, by definition, the centres of rotation render the uprights or bars capable of rotating in only one plane about two parallel axes, allowing transversal thrusts to be created only by means of specific filler elements.

The articulated joint according to the invention envisages that the two centres of rotation of the uprights or bars are in turn fixed on a monocentric system that thus removes the constraint of parallelism of the rotation axes, with the possibility of creating angular bending relative to the rotation plane and of creating transversal thrusts relative to the plane of angular movement of the joint, thereby producing a structure such as to be able to be used for the correction of axial deviations of the joints.

An additional feature offered by the present invention is that it comprises means enabling the angular strokes of the uprights or bars to be adjusted both relative to the respective centres of rotation and relative to the plane of angular movement of the joint, so as to lock the uprights or bars in predetermined positions.

The present invention has application in the medical and orthopaedic fields, in particular in the manufacture of braces in general, as well as prostheses and braces mainly for use in conservative, post-traumatic, rehabilitative and postoperative therapy.

### PRIOR ART

It is well known that subjects presenting with orthopaedic problems of the knee joints, but similarly of other joints as well, such as the ankle or elbow joints, above all in the case of injuries or as a consequence of a previous surgical procedure, need to use an orthopaedic brace, or orthosis, designed to ensure or control the function of a hinged constraint between the femur and tibia or other lever pivot points of joints, by supporting stresses that would otherwise be damaging for the joint itself.

The function of an orthosis is, in general, to ensure a relative immobilisation or limitation of a joint affected by injuries or arthrosis or ligament sprains or which has undergone a surgical procedure.

Another use of an orthosis is in conjunction with functional rehabilitation or re-education, where the orthosis can be used to reduce the load on a joint and decrease its pain, or be adopted for preventive purposes in cases of osteoporosis or bone yielding.

An orthosis usually consists of a rigid or soft frame encircling the limb and designed to ensure an adequate harnessing of the joint with the aim of preventing the occurrence of stress on the ligaments or synovial membranes when the injured and/or convalescing individual is walking.

According to the prior art, the frame of an orthosis for joints, for example in the typical case of a knee brace, comprises means of constraint to the femur and tibia and a section connecting said means, consisting of an articulated joint positioned at the level of the knee itself.

The means of constraint usually consist of uprights or bars that are fixed by suitable straps which wrap around both the femur and tibia of the injured subject, or else bands, straps or sleeves made of fabric which enable a constraint to be created between the end of the mechanical articulated joint and the limb.

The mechanical articulated joint is positioned laterally relative to the femur and tibia, in the case of an example of application on the lower limb. Thanks to its configuration, consisting of two centres of rotation relatively constrained to each other thanks to toothed profiles, said articulated joint follows the kinematics of the knee with good approximation.

A standard polycentric articulated joint is a system made up of two hinged uprights or bars, each with its own centre of rotation, wherein said centres of rotation are constrained to each other in a parallel fashion and at a known distance.

In a standard polycentric articulated joint, the two uprights or bars are connected to each other thanks to a circular toothed profile present on each bar, and the toothed profile sets them in synchronous motion relative to each other.

In said articulated joint, by definition, the centres of rotation and the toothed profile make the uprights or bars able to rotate only about two parallel axes and their rotation motion thus lies in a common plane.

The problems to which a joint may be subject include varus and valgus, which are skeletal anomalies identifiable in axial deviations of the limbs, where the skeletal segments, situated distally to the portion in which the deviation occurs, are in an anomalous position of abduction in varus and adduction in valgus, that is, in the former case farther and in the latter case closer to the midline of the body.

A typical case of varus-valgus regards the knees which, if suffering from varus, tend to be bent outwards from the body relative to an undeformed axis of the limb. In situations of valgus, by contrast, the knees tend to be bent inwards, reciprocally near each other.

In both of the anomalies described above, the subject tends to experience difficulties in walking, which are greater the smaller the obtuse angle is, be it open inwards as in the case of varus or open outwards as in the case of valgus.

Various solutions in this regard are known in the art, for example through the use of special knee supports and braces intended to correct any misalignment of the joint, generally through the use of spacers of various kinds inserted into the brace in proximity to the lateral thrust zone to be applied on the articulated joint.

Though they at least partially resolve the limb deformations, such solutions are not devoid of problems, substantially tied to the impractical and unstable use of the orthosis.

Document US 2018/036159 A1 discloses an articulated joint which comprises a platform for resting upon a part of the body to be treated, such as, for example, the knee, elbow, or other parts, associated with which there are two bodies adapted to receive the longitudinal ends of respective bars or uprights that are in turn fixable to the part of the body to be treated, wherein said bars or uprights are respectively connected to said two bodies by means of respective rotation pins.

Said bodies are pivotable in rotation relative to the respective longitudinal axes of the rotation pins.

According to this solution said two bodies bearing the bars are reciprocally and correspondingly hinged onto said platform by means of a common pin, slidably applied on which there is a slider with circular peripheral toothing adapted to connect the reciprocal primary angular movement of said two bars or uprights.

Furthermore, each of said bars or uprights possesses a toothed end cooperating with said slider, so that said common pin allows a secondary reciprocal angular movement between said two bodies according to a circular axis centred on the secondary rotation axis of the common pin and tangent to the two primary rotation axes of the bars.

This solution, despite increasing the versatility of the articulated joint, since added to the primary rotation angle relative to the respective longitudinal axes of the rotation pins there are secondary reciprocal angular movements between said two bodies according to a circular axis centred on the secondary rotation axis of the common pin and tangent to the two primary rotation axes of the uprights or bars, it is nonetheless limited due to the fact that the operating angles are difficult to adjust, particularly during use; in fact, a gradual positioning of the limb requires a continuous measurement of the obtuse angle between the femur and tibia, in the case of the knee, with a consequent adaptation of the orthosis, especially as regards the rigid frame thereof.

Another drawback is represented by the fact that such braces are sometimes difficult to fit, since they consist of a number of reciprocally connected portions that can be fixed by means of any suitable temporary locking means.

Document WO 01/21114 A1 discloses a hinge with micrometric angular adjustment that comprises a first hinge element and a second hinge element coupled by means of a first cylindrical pivot that is rotatable about its longitudinal axis; said first cylindrical pivot is fixed to the first hinge element, and it is provided with a series of teeth which are radially disposed adjacent to each other, and which are made on the side surface of the central portion of said first pivot. Furthermore, the hinge comprises a second pivot that is fixed to said second hinge element in such a way as it is rotatable about its longitudinal axis that is perpendicular to the axis of said first pivot. Finally, the side surface of said second pivot is provided with a series of endless screw threads suitable for cooperating with the teeth of said first pivot in such a way that a rotation of said second pivot (40) about its axis (B) results in a rotation of said first pivot about its axis (A).

### DESCRIPTION OF THE INVENTION

The present invention aims to provide a polycentric articulated joint for orthopaedic orthoses or braces designed for rehabilitation of a knee or other orthopaedic braces applicable as aids for joints of the human body, such as an ankle, an elbow or the like, which is capable of eliminating or at least reducing the drawbacks highlighted above.

Said objective is achieved through the use of an articulated joint for orthopaedic orthoses or braces designed for rehabilitation of joints which is produced with a conception aimed at the possibility that, in addition to the removal of the constraint of the parallelism of the axes, there are means of adjusting and locking the varus-valgus angle in order also to treat cases in which the joint develops a natural varus or valgus angle, said means envisaging the use of a nut mounted on a body of the articulated joint, which engages on a toothed profile fashioned (directly or applied) on another body of the articulated joint.

In this manner, one obtains the possibility of a predeterminable and fixable adjustment, as desired, of the angular movement of both uprights or bars relative to a common neutral plane that contains them.

This is obtained by means of an articulated joint for orthopaedic orthoses or braces designed for rehabilitation of a limb, whose features are described in claim 1.

The dependent claims of the solution in question outline advantageous embodiments of the invention.

The main advantages of this solution regard first of all the fact that the articulated joint in its entirety, compared to the prior art solutions, is based on a construction method of a biaxial polycentric type, wherein the two centres of rotation of the uprights or bars are fixed on a monocentric system that removes the constraint of parallelism between the rotation axes, and which comprises the possibility of adjusting and locking the angle of angular movement of the uprights or bars relative to a common plane that contains them, which can be predetermined according to need.

### ILLUSTRATION OF THE DRAWINGS

Other features and advantages of the invention will become more apparent on reading the following description of an embodiment of the invention, provided by way of nonlimiting example with the aid of the drawings illustrated in the attached figures, in which:

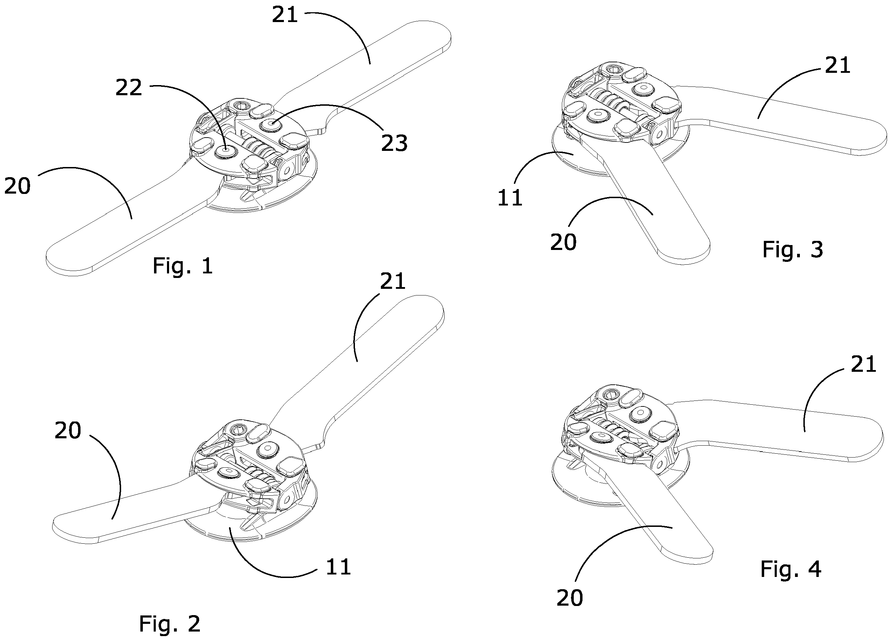

- figures 1 to 4 represent schematic views of the articulated joint according to the invention in four different operating phases using a monocentric system that removes the constraint of parallelism between the axes, with the uprights or bars being able to rotate about a common centre, and comprising a system provided with means for adjusting the angle of angular movement of the uprights or bars relative to a common plane that contains them;
- figure 5 illustrates a schematic side view of the articulated joint, according to the invention, highlighting the possibility of rotation of the bars about the primary centre of rotation with the addition of means for adjusting the angle between the uprights or bars relative to a common plane that contains them;
- figure 6 is a schematic exploded axonometric perspective view of the articulated joint according to the invention;

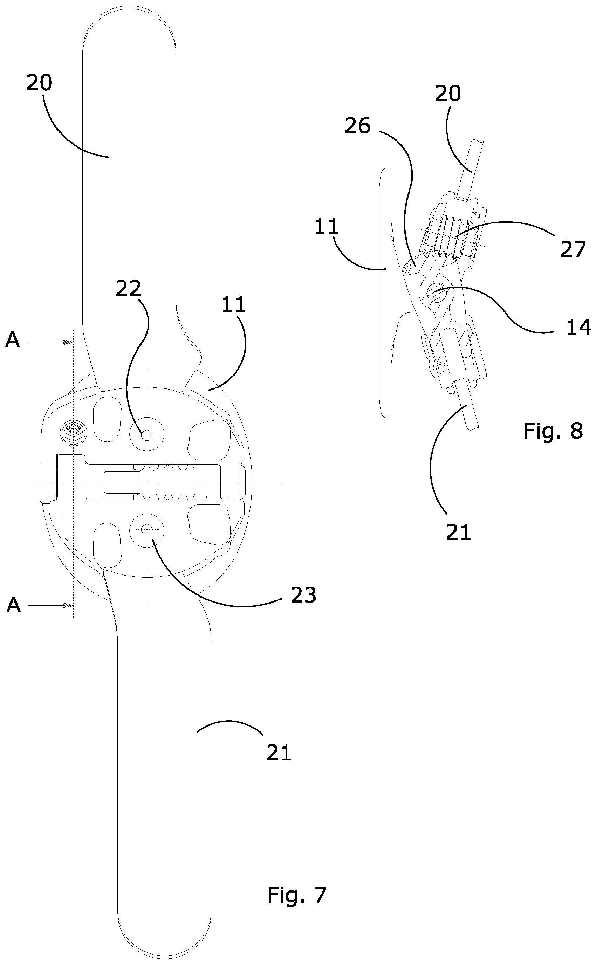

- figure 7 is a schematic view of the articulated joint according to the invention represented frontally with the uprights or bars inclined according to an angle relative to a common plane that contains them in a neutral position, with the obtuse angle facing outward;
- figure 8 is a schematic and detailed sectional view of the articulated joint according to the invention according to the lines A-A of figure 7.

### DESCRIPTION OF AN EMBODIMENT OF THE INVENTION

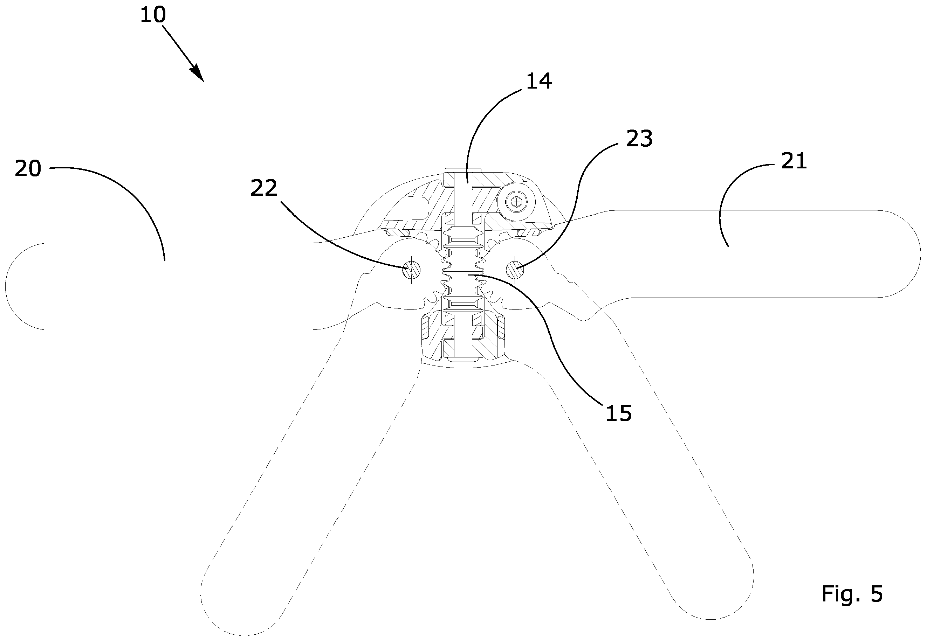

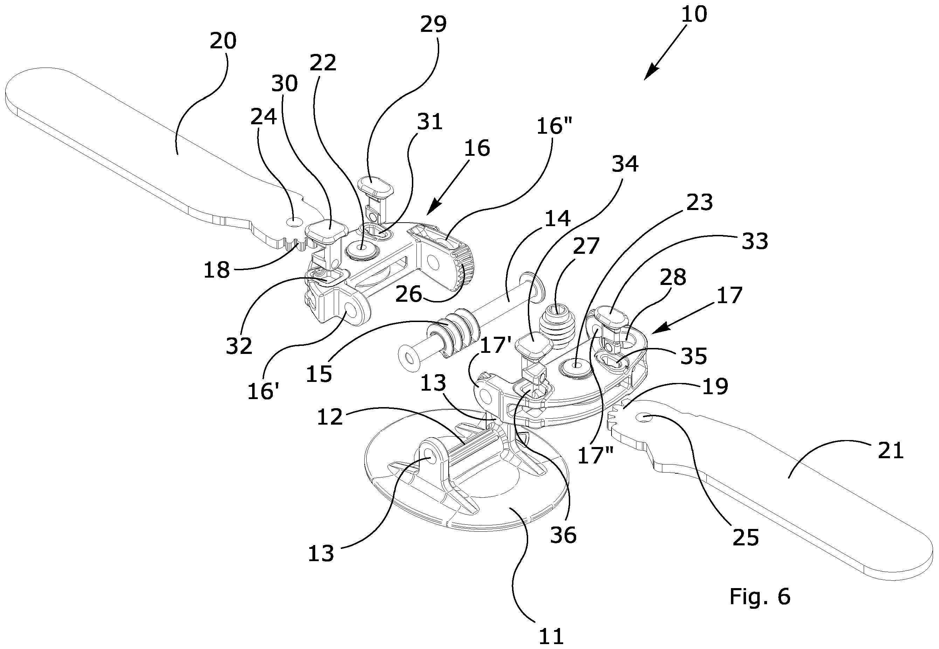

Making reference initially to figures 5 and 6, the number 10 denotes in its entirety a biaxial polycentric articulated joint for joints, with controllable angular movement.

The articulated joint 10 according to the invention comprises a rest platform 11 for resting on the part of the body to be treated, such as, for example, the knee, or elbow, or other parts, which represents the condyle support adapted to distribute the thrust effect on the condyle of the joint to be treated.

According to the embodiment represented in figures 5 and 6, the platform 11 is provided with a support structure 12, facing on the opposite side relative to the one of the condyle support, which comprises two diametrically opposite forks, fashioned on which there are respective holes 13 adapted to receive a articulation pin 14, slidably mounted along whose shaft there is an axially perforated cylindrical slider 15, fashioned on which there are circular toothings, designed for the purposes that will be described below.

On the support structure 12, and in particular on the articulation pin 14, two bodies are applied, indicated as 16 and 17, which have a substantially semi-circular or half-moon shape and are disposed correspondingly relative to the pin 14, and each of them comprises perforated fork-like projections, indicated as 16' and 16" for the body 16 and 17' and 17" for the body 17, said fork-like projections being adapted to be engaged onto the pin 14.

Since the two bodies 16 and 17 are reciprocally and correspondingly pivoted on the pin 14, they are able to carry out angular strokes relative to a common plane that contains them, in a neutral position, for the adjustment of the varus-valgus positioning of the articulated joint, as will be described below.

Each of the two bodies 16 and 17 has a slot obtained from a cut made along the whole arch of a circle and on the centre line of the medial plane, and which terminates in the inner wall of each body at an opening that leads towards the centre of the articulated joint in proximity to the slider 15 of said pin 14.

Said slots of the two bodies 16 and 17 are adapted to receive toothed longitudinal ends 18 and 19 of respective bars or uprights 20 and 21.

The bars or uprights 20 and 21 are in turn fixable, during use, to a part of the body of a patient to be treated, such as the femoral and tibial sector in the case of a leg, or humeral and ulnar sector of an arm, or other joints to be treated, possibly after the interposition of a suitable orthosis.

Furthermore, each of the two bodies 16 and 17 has a transversal hole fashioned in the centre plane, inserted into which there are respective pins 22 and 23 that intercept respective holes 24 and 25 fashioned on the uprights or bars 20 and 21 and represent the first hinge of the bars themselves.

The pins 22 and 23 represent the two corresponding symmetric rotation axes of the two bars 20 and 21; the axes are parallel in the event that the bars are disposed in the same common plane, i.e. at a 180° angle relative to each other, but become variable since the bars 20 and 21 themselves are located on the two bodies 16 and 17 subject to an angular adjustment on the axis of the pin 14, in order to obtain adjustments of the varus-valgus angle.

According to the embodiment represented in figures 5 and 6, one of the two perforated fork-like projections of the body 16, namely, the one indicated as 16", projects relative to the other and fashioned on the end facing towards the body 17 there is a fixed toothed sector 26 exhibiting a curvature having a radius whose centre is located on the axis of rotation of the two bodies 16 and 17 and thus on the pin 14.

During use, i.e. when the two bodies 16 and 17 are connected to the common pin 14, said fixed toothed sector 26 penetrates into the body 17 for a certain length so as to reach a rotating nut with internal teeth 27, located captive in a seat 28 thereof present in the body 17.

The nut with internal teeth 27 is disposed on a transversal axis relative to the surface plane of the articulated joint body 17 in which it is inserted, and has a shaped seat into which an adjustment tool can be introduced in order to adjust the working angle between the two uprights or bars 20 and 21 relative to the common plane that contains them in a neutral position, with variable positioning depending on whether the correction must be on the vagus or valgus.

The position of the nut with internal teeth 27 is tangent relative to the fixed toothed sector 26 and the rotation thereof, driven by a tool, determines the variation of the adjustment angle of the two bodies 16 and 17 and consequently also that of the planes in which the uprights or bars 20 and 21 lie relative to the common plane that contains them in a neutral position, thanks to the rotation relative to the axis of the pin 14, for the adjustment of the varus-valgus angle of the articulated joint.

Furthermore, according to one embodiment of the invention again represented in figures 5 and 6, the articulated joint system according to the invention comprises a second adjustment system whereby it is possible to set the bending and extension angles of each upright or bar 20 and 21.

In particular, said second adjustment system comprises pairs of inserts insertable transversally into specific seats fashioned on each of the bodies 16 and 17, said inserts being suitable for intercepting and locking in a pre-established position the bending or extension angle of the uprights or bars 20 and 21 relative to the centres of rotation thereof located on the axes of the holes 24 and 25, i.e. of their pins 22 and 23.

More precisely, said second adjustment system comprises inserts 29 for locking the extension angle and 30 for locking the bending angle which are insertable into respective holes 31 and 32 fashioned on the body 16, in a transversal direction relative to the surface thereof, so as to intercept and lock in a pre-established position the bending or extension angle of the upright or bar 20 relative to the centre of rotation thereof located on the axis of the hole 24 and the pin 22 thereof.

Similarly, said second adjustment system comprises inserts 33 for locking the extension angle and 34 for locking the bending angle which are insertable into respective holes 35 and 36 fashioned on the articulated joint body 17 in a transversal direction relative to the surface thereof, so as to intercept and lock in a pre-established position the bending or extension angle of the upright or bar 21 relative to the centre of rotation thereof located on the axis of the hole 25 and the pin 23 thereof.

In order to perform the bending or extension adjustment of the uprights or bars 20 and 21 on the respective rotation axes, use is made of said inserts 29 and 30 insertable into the body 16 and 33 and 34 insertable into the body 17. The extension or bending angle of the uprights or bars 20 and 21 is adjusted while the inserts are not inserted, and once the extension or bending position established by the operator has been reached, all of the inserts are inserted into the respective seats 31 and 32 on one side and 35 and 36 on the other side, thus intercepting the toothed longitudinal ends 18 and 19 and establishing the rotational locking of the bars in the set position.

From an operational viewpoint, and summing up, the articulated joint according to the invention comprises two (primary) centres of rotation created on a common plane by the pins 22 and 23 of the uprights or bars 20 and 21 which rotate, and a monocentric (secondary) system defined by the pin 14, which removes the constraint of parallelism between the axes of the uprights or bars, which can move angularly like "flapping wings", whilst the circular toothed slider 15, which slides along the pin 14 and defines the axis of the secondary centre of rotation, enables the uprights or bars to remain connected to each other also when they rotate about secondary centre of rotation, effectively becoming detached from the common plane in which the uprights or bars lie in the neutral position.

According to different aspects of the invention, there is envisaged the advantageous use of a rotating nut with internal teeth 27 and inserts 29 and 30 on one side and 33 and 34 on the other, which determine, respectively, the variation of the adjustment angle of the two bodies 16 and 17 and consequently of the uprights or bars 20 and 21 relative to the axis of the pin 14, for the respective adjustments and locking, on the one hand, of the varus-valgus angle of the articulated joint, and, on the other hand, of the bending or extension angle of the uprights or bars 20 and 21 on the respective rotation axes, with the aim of constraining the rotation angle of said uprights or bars on both the primary axis and secondary axis.

## Claims

1. An articulated joint (10) for orthopaedic orthoses or braces comprising a rest platform (11), two bodies (16, 17) connected to said platform (11), each of which is adapted to receive a longitudinal end provided with peripheral toothing (18, 19) of a respective bar or upright (20, 21), each bar or upright (20, 21) being respectively connected by means of a respective rotation pin (22, 23), to one of said two bodies (16, 17) and being pivotable in rotation relative to a respective longitudinal axis of the rotation pin (22, 23), each of said two bodies (16, 17) being hinged onto said platform (11) by means of a common pin (14) slidably applied on which there is a slider (15) with peripheral toothing adapted to cooperate with the peripheral toothing of each respective bar or upright (20, 21), **characterised in that** one (16) of said bodies (16, 17) comprises a first circular sector-shaped element (16") provided with peripheral toothing (26) and pivotable about the longitudinal axis of said common pin (14), **in that** the other (17) of said bodies (16, 17) comprises a second rotating nut-shaped element (27) provided with internal teeth, **in that** said first element (16") and said second element (27) cooperate with each other by means of the respective teeth so as to allow a synchronised rotation of the two bodies (16, 17) and thus of the two bars or uprights (20, 21) about the longitudinal axis of said common pin (14).

2. The articulated joint (10) according to claim 1, **characterised in that** said rest platform (11) comprises a support structure (12) for the common pin (14), on which the two bodies (16, 17) are pivotably applied.

3. The articulated joint (10) according to the preceding claims, **characterised in that** each of the two bodies (16, 17) comprises a pair of perforated ends (16', 16"; 17', 17") inside which the common pin (14) is disposed.

4. The articulated joint (10) according to the preceding claims, **characterised in that** the second rotating nut-shaped element (27) provided with internal teeth is located captive in a seat (28) of the other (17) of the two bodies (16, 17) and has a shaped seat that may be accessed by means of a tool in order to adjust the rotation angle of the bodies (16, 17) and thus of the bars or uprights (20, 21) about the longitudinal axis of the common pin (14).

5. The articulated joint according to one of the preceding claims, **characterised in that** each of said bodies (16, 17) has at least one respective seat (31, 32; 35, 36) adapted to receive a respective insert (29, 30; 33, 34) which is adapted to intercept the rotational movement of each bar or upright (20, 21) about the respective rotation pin (22, 23) and to prevent said rotational movement beyond a predetermined angle.

6. The articulated joint according to claim 5, **characterised in that** said respective insert (29, 30; 33, 34) intercepts the rotational movement of each bar or upright (20, 21) at the peripheral toothing (18, 19) of each bar or upright (20, 21).

## Patentansprüche

1. Gelenk (10) für orthopädische Orthosen oder Bandagen, das eine Auflageplattform (11), zwei mit der Plattform (11) verbundene Körper (16, 17), die jeweils geeignet sind, um ein mit einer Umfangsverzahnung (18, 19) versehenes Ende eines Stabs oder Trägers (20, 21) aufzunehmen, umfasst, wobei jeder Stab oder Träger (20, 21) jeweils durch einen jeweiligen Rotationsbolzen (22, 23) mit einem der zwei Körper (16, 17) verbunden ist und in Bezug auf eine jeweilige Längsachse des Rotationsbolzens (22, 23) in Rotation schwenkbar ist, wobei jeder der beiden Körper (16, 17) an der Plattform (11) durch einen gemeinsamen Bolzen (14) angelenkt ist, auf dem ein Schieber (15) mit einer Umfangsverzahnung verschiebbar angebracht ist, die geeignet ist, mit der Umfangsverzahnung von jedem jeweiligen Stab oder Träger (20, 21) zusammenzuwirken, **dadurch gekennzeichnet, dass** einer (16) der Körper (16, 17) ein erstes kreissektorförmiges Element(16") umfasst, das mit Umfangsverzahnung (26) ausgestattet und um die Längsachse des gemeinsamen Bolzens (14) schwenkbar ist, dass der andere (17) der Körper (16, 17) ein zweites, rotierendes, mutterförmiges Element (27) umfasst, das mit Innenzähnen versehen ist, dass das erste Element (16") und das zweite Element (27) mithilfe der jeweiligen Zähne zusammenwirken, um eine synchronisierte Rotation der zwei Körper (16, 17) und auf diese Weise der beiden Stäbe oder Träger (20, 21) um die Längsachse des gemeinsamen Bolzens (14) zu ermöglichen.

2. Gelenk (10) nach Anspruch 1, **dadurch gekennzeichnet, dass** die Auflageplattform (11) eine Stützstruktur (12) für den gemeinsamen Bolzen (14) umfasst, an dem die zwei Körper (16, 17) schwenkbar angebracht sind.

3. Gelenk (10) nach den vorausgehenden Ansprüchen, **dadurch gekennzeichnet, dass** jeder der zwei Körper (16, 17) ein Paar perforierte Enden (16', 16"; 17', 17") aufweist, in denen der gemeinsame Bolzen (14) angeordnet ist.

4. Gelenk (10) nach den vorausgehenden Ansprüchen, **dadurch gekennzeichnet, dass** das zweite, sich drehende, mutterförmige Element (27), das mit Innenzähnen versehen ist, unverlierbar in einem Sitz (28) des anderen (17) der zwei Körper (16, 17) angeordnet ist und einen geformten Sitz aufweist, der mithilfe eines Werkzeugs zugänglich ist, um den Rotationswinkel der Körper (16, 17) und auf diese Weise der Stäbe oder Träger (20, 21) um die Längsachse des gemeinsamen Bolzens (14) einzustellen.

5. Gelenk nach einem der vorausgehenden Ansprüche, **dadurch gekennzeichnet, dass** jeder der Körper (16, 17) mindestens einen jeweiligen Sitz (31, 32; 35, 36) aufweist, der geeignet ist, einen jeweiligen Einsatz (29, 30; 33, 34) aufzunehmen, der geeignet ist, die Rotationsbewegung von jedem Stab oder Träger (20, 21) um den jeweiligen Rotationsbolzen (22, 23) abzufangen und die Rotationsbewegung über einen vorgegebenen Winkel hinaus zu verhindern.

6. Gelenk nach Anspruch 5, **dadurch gekennzeichnet, dass** der jeweilige Einsatz (29, 30; 33, 34) die Rotationsbewegung von jedem Stab oder Träger (20, 21) an der Umfangsverzahnung (18, 19) von jedem Stab oder Träger (20, 21) abfängt.

## Revendications

1. Articulation (10) pour orthèses ou attelles orthopédiques comprenant une plateforme de repos (11), deux corps (16, 17) connectés à ladite plateforme (11), dont chacun est adapté à recevoir une extrémité longitudinale pourvue d'une denture périphérique (18, 19) d'une barre ou montant respectif (20, 21), chaque barre ou montant (20, 21) étant respectivement connecté au moyen d'une tige de rotation respective (22, 23), à l'un desdits deux corps (16, 17) et pouvant être pivoté en rotation par rapport à un axe longitudinal respectif de la tige de rotation (22, 23), chacun desdits deux corps (16, 17) étant articulé sur ladite plateforme (11) au moyen d'une tige commune (14) sur laquelle est appliqué de manière à pouvoir coulisser un élément coulissant (15) pourvu d'une denture périphérique adaptée à coopérer avec la denture périphérique de chaque barre ou montant respectif (20, 21), **caractérisé en ce que** l'un (16) desdits corps (16, 17) comprend un premier élément circulaire en forme de secteur (16") pourvu d'une denture périphérique (26) et pouvant être pivoté autour de l'axe longitudinal de ladite tige commune (14), **en ce que** l'autre (17) desdits corps (16, 17) comprend un second élément rotatif en forme d'écrou (27) pourvu de dents internes, **en ce que** ledit premier élément (16") et ledit second élément (27) coopèrent l'un avec l'autre au moyen des dents respectives pour permettre une rotation synchronisée des deux corps (16, 17) et par conséquent des deux barres ou montants (20, 21) autour de l'axe longitudinal de ladite tige commune (14).

2. Articulation (10) selon la revendication 1, **caractérisée en ce que** ladite plateforme de repos (11) comprend une structure de maintien (12) pour la tige commune (14), sur laquelle les deux corps (16, 17) sont appliqués de manière à pouvoir pivoter.

3. Articulation (10) selon les revendications précédentes, **caractérisée en ce que** chacun des deux corps (16, 17) comprend une paire d'extrémités perforées (16', 16" ; 17', 17") à l'intérieur desquelles est disposée la tige commune (14).

4. Articulation (10) selon les revendications précédentes, **caractérisée en ce que** le second élément rotatif en forme d'écrou (27) pourvu de dents internes est situé de façon captive dans un siège (28) de l'autre (17) des deux corps (16, 17) et comprend un siège profilé accessible au moyen d'un outil pour régler l'angle de rotation des corps (16, 17) et par conséquent des barres ou montants (20, 21) autour de l'axe longitudinal de la tige commune (14).

5. Articulation selon l'une des revendications précédentes, **caractérisée en ce que** chacun desdits corps (16, 17) présente au moins un siège respectif (31, 32 ; 35, 36) adapté à recevoir un insert respectif (29, 30 ; 33, 34) qui est adapté à intercepter le mouvement rotationnel de chaque barre ou montant (20, 21) autour de la tige de rotation respective (22, 23) et à empêcher ledit mouvement rotationnel au-delà d'un angle prédéterminé.

6. Articulation selon la revendication 5, **caractérisée en ce que** ledit insert respectif (29, 30 ; 33, 34) intercepte le mouvement rotationnel de chaque barre ou montant (20, 21) au niveau de la denture périphérique (18, 19) de chaque barre ou montant (20, 21).
